# EUROPEAN PATENT APPLICATION

(11) **EP 3 705 094 A1**
(43) Date of publication of application: **09.09.2020**
(21) Application number: 20161560.6
(22) Date of filing: 06.03.2020
(51) Int. Cl.: A61F 2/52, A61F 13/14, A61F 2/78

(54) **PROSTHESIS AND METHOD FOR APPLYING SUCH A PROSTHESIS**

(30) Priority: 08.03.2019 NL 2022694
(71) Applicant: 2be Yourself Beheer B.V., 3319 EA Dordrecht (NL)
(72) Inventor: Klink, Teunis Floris, 3319 EA Dordrecht (NL)
(74) Representative: Arnold & Siedsma

(57) **Abstract**

The present disclosure relates to a prosthesis, comprising a basis having an engagement surface configured to engage a chest portion with a wound area of a user; and comprising an emulator configured to be releasably connected to the basis and comprising an outer contour emulating a breast of the user, wherein the engagement surface of the basis comprises a shape that substantially complements the wound area to transfer a therapeutic compressive force to the wound area. The present disclosure furthermore relates to a method for applying a prosthesis in accordance with the present disclosure, said method comprising providing a basis having an engagement surface configured to engage a chest portion with a wound area of a user, wherein the engagement surface of the basis comprises a shape that substantially complements the wound area to transfer a therapeutic compressive force to the wound area; and providing an emulator configured to be releasably connected to the basis and comprising an outer contour emulating a breast of the user.

## Description

The present disclosure relates to a prosthesis, in particular a breast prosthesis, and to a method for applying such a prosthesis. Prostheses of the aforementioned type are typically applied following a mastectomy, wherein one or both of the female breasts are partially or fully removed as treatment for, or in some cases as a preventive measure against, breast cancer.

During usage, such breasts prostheses are carried outside the body by a user at the former location of an amputated breast, thereby emulating said amputated breast. Prostheses are available in a number of different standardised sizes, which may or may not match a remaining and/or the amputated breast in terms of size and shape to a degree that is considered acceptable.

Furthermore, during a recovery process following a mastectomy, patients are typically issued therapeutic compression means, such as a compressive garment, to exert a therapeutic compressive force on the wound area resulting from the mastectomy. The application of such a therapeutic compressive force, known as compression therapy, is known to assist in preventing complications, such as edema, following the mastectomy as well as in promoting an optimal healing process of the wound area. The length of the recovery period following a mastectomy is approximately two years, during which it is not possible for a user to use a prosthesis of the aforementioned type due to the presence of the compressive garment. Moreover, during the recovery process the wound area undergoes constant changes due to the formation of scar tissue and possibly the occurrence of edema, seroma or radiation fibrosis, which may result in a shifting of pain points on the wound area and a need to replace the compressive garment.

Reference is made here to JP H04 79950 A, which is considered to constitute the closest prior art. JP H04 79950 A discloses an artificial breast of which a back face comprises fasteners to be attached to a front surface of a base member. The base member moreover comprises a bonding surface opposite the front surface to be attached to the chest of a patient by means of an adhesive.

The prosthesis disclosed by the aforementioned closest prior art comprise the disadvantage that it cannot be used during a recovery period following a mastectomy, in which it is generally required to apply a therapeutic compression means to exert a therapeutic compressive force on the wound area resulting from a mastectomy to promote optimal healing of the wound area.

Moreover, publications US 6 156 065 A; US 2010/023123 A1; and US 7 058 439 B2 are acknowledged here to constitute further prior art.

An objective of the present invention is to provide a prosthesis and a method for applying such a prosthesis that is configured to be used during a recovery period following a mastectomy during which a therapeutic compressive force must be exerted on the wound area that is subject to change due to healing of the wound area.

This objective is achieved with a prosthesis according to the present invention, comprising a basis having an engagement surface configured to engage a chest portion with a wound area of a user; and an emulator configured to be releasably connected to the basis and comprising an outer contour emulating a breast of the user, wherein the engagement surface of the basis comprises a shape that substantially complements the wound area to transfer a therapeutic compressive force to the wound area. Due to the prosthesis comprising a basis and an emulator configured to be releasably connected to the basis, a greater degree of flexibility is achieved wherein the advantages of a prosthesis and a transfer of the therapeutic compressive may be combined or further improved.

In preferred embodiments of a prosthesis according to the present invention, the shape of the engagement surface of the basis that complements the wound area is based on a three-dimensional scan of the wound area, and moreover comprises at least one of a recess and a protrusion relative to the complementary shape of the engagement surface to define a predetermined therapeutic pressure profile to be transferred to, and thereby exerted on, the wound area.

In these preferred embodiments of the prosthesis in accordance with the present invention, the aforementioned therapeutic effect stemming from the engagement surface is further improved in that said engagement surface transfers, by means of the at least one protrusion, a (relatively high) therapeutic pressure to specific regions of the wound area that require exertion of such a pressure to promote healing. Such areas of the wound area may include areas susceptible to local secondary lymphedema, postoperative seroma and radio-induced fibrosis. Simultaneously, said engagement surface transfers, by means of the at least one recess, a relatively low or absent therapeutic pressure to regions of the wound area that cannot bear the magnitude of therapeutic pressure exerted elsewhere on the wound area or even any pressure. Such areas may include areas including birthmarks, prominent scar tissue and hypersensitive skin portions.

The present disclosure furthermore relates to a method for applying a prosthesis of the type described above, the method comprising providing a basis having an engagement surface configured to engage a chest portion with a wound area of a user, wherein the engagement surface of the basis comprises a shape that substantially complements the wound area to transfer a therapeutic compressive force to the wound area; and providing an emulator configured to be releasably connected to the basis and comprising an outer contour emulating a breast of the user.

In preferred embodiments of the method for applying a prosthesis in accordance with the present invention, the step of providing the basis further comprises performing a three-dimensional scan of the wound area of the chest portion of the user to obtain a digital model of the wound area. In said digital model of the wound area, at least one of an area requiring a decreased or absent therapeutic pressure to be transferred thereto, and an area requiring an increased therapeutic pressure to be transferred thereto may be identified. Subsequently, the engagement surface of the basis is formed to comprise one of a recess corresponding to the area requiring a decreased therapeutic pressure to be exerted thereon, and a protrusion corresponding to the area requiring an increased therapeutic pressure to be exerted thereon.

As such, by means of the above preferred method, a prosthesis in accordance with the present invention is provided with which a relatively high therapeutic pressure is transferred to specific regions of the wound area to promote healing, and simultaneously a relatively low or no therapeutic pressure is transferred to other specific areas of the wound area that are sensitive to having pressure applied thereon. Consequently, there is provided a prosthesis that combines maximum therapeutic effect with a high wearing comfort during a recovery period following surgery. Because the emulator of the prosthesis is moreover releasably connected to the basis, said basis may be periodically replaced to account for spatial shifts of sensitive and to-be-pressured regions of the wound area.

The invention further relates to a kit of parts, comprising a basis and an emulator of the prosthesis according to the present invention. In further preferred embodiments, the kit-of-parts moreover comprises a tensioner.

Preferred embodiments of the prosthesis, the method for applying such a prosthesis and a kit-of-parts in accordance with the present invention are subject of the appended dependent claims.

The various aspects and features described and shown in the present disclosure can be applied, individually, wherever possible. These individual aspects, and in particular the aspects and features described in the attached dependent claims, may be made subject of divisional patent applications.

In the following description preferred embodiments of the present invention are further elucidated with reference to the drawing, in which:
Figure 1 shows a chest portion of a user prior to mastectomy;
Figure 2 shows a chest portion of a user following a mastectomy, wherein one of the breasts shown in Figure 1 is removed;
Figure 3 shows a side view of a prosthesis in accordance with the present invention, comprising a basis and an emulator;
Figure 4 shows a view of a back surface of the emulator shown in Figure 3, when viewed from direction IV as shown in Figure 3;
Figure 5 shows a view of a frontal surface of the basis shown in Figure 3, when viewed from direction V as shown in Figure 3;
Figure 6 shows a view of an engagement surface of the basis shown in Figure 3, when viewed from direction VI as shown in Figure 3;
Figure 7 illustrates a process of applying the breast prosthesis of Figure 3 in accordance with the method according to the present invention;
Figure 8 illustrates the breast prosthesis of Figure 3 in an applied state;
Figure 9 illustrates a process of applying the breast prosthesis of Figure 3 in conjunction with a user in accordance with the method according to the present invention;
Figure 10 illustrates the breast prosthesis of Figure 3 in an applied state in conjunction with a user.
Figure 11 shows the breast prosthesis of Figure 10 in conjunction with a tensioner; and
Figure 12 depicts a digital model of a wound area following a mastectomy upon which an engagement surface of the emulator in accordance with the present invention may be based.

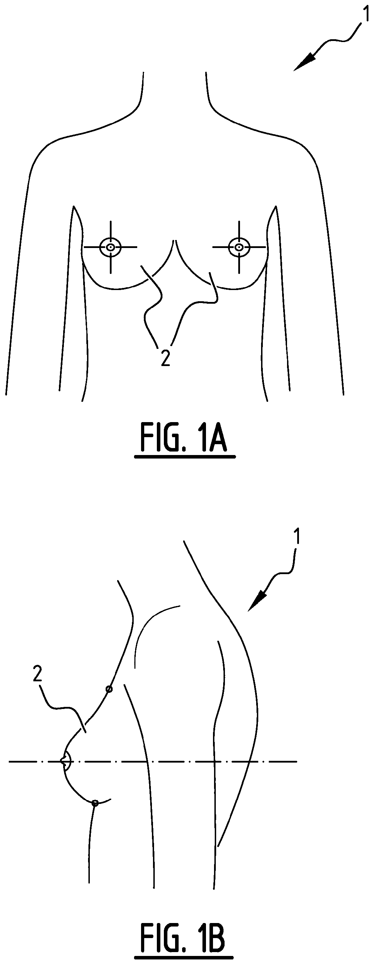

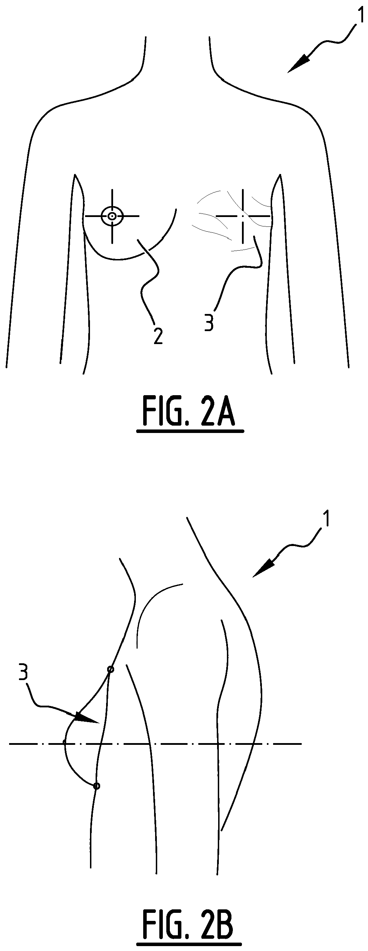

In Figures 1A and 1B, a user 1 having breasts 2 is shown in a state prior to a mastectomy. In Figures 2A and 2B, user 1 is shown in a state following a mastectomy, wherein one breast 2 has been surgically removed, resulting in a wound area 3 on a portion of the chest of user 1.

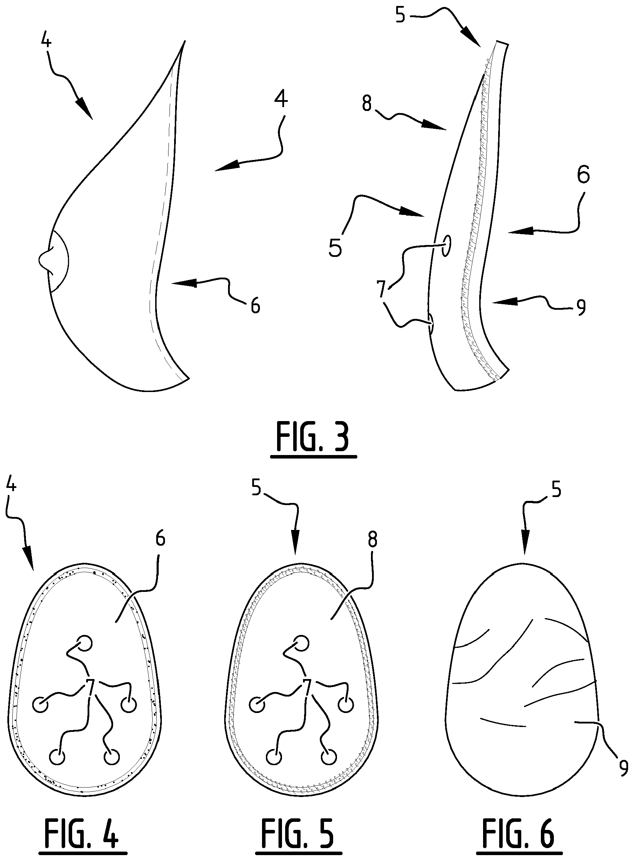

Figure 3 shows a prosthesis in accordance with the present invention. The prosthesis comprises an emulator 4 having an outer contour emulating a shape of breast 2 of user 1. In preferred embodiments of the present invention, the outer contour of emulator 4 is based on a three-dimensional (3D) scan of breast 2 having been performed prior to the surgical removal of breast 2. Alternatively, the outer contour of emulator 4 may be based on a 3D scan of a remaining breast 2 that has not been surgically removed through performing a mastectomy. The 3D scan of breast 2 with or without breast 2 being covered by a bra. Moreover, said 3D scan may be performed with any of the suitable scanning techniques known to the skilled person, with scanning techniques based on time-of-flight and triangulation mentioned here solely as examples. Emulator 4 may furthermore emulate breast 2 by comprising a weight that approximates at least a portion of the weight of surgically removed breast 2 and may be manufactured from a material that resembles a breast 2 in terms of elasticity, density or skin colour. When viewed from direction IV, emulator 4 may furthermore comprise a back surface 6 at a side facing away from the breast-shaped outer contour of emulator 4.

The prosthesis as shown in Figure 3 furthermore comprises a basis 5 having a frontal surface 8 visible when viewed from direction V and an engagement surface 9 visible when viewed from direction VI. In accordance with the present invention, engagement surface 9 is configured to engage a chest portion of user 1, in particular a chest portion comprising wound area 3. Moreover, emulator 4 may be releasably connected to basis 5 by placing back surface 6 of emulator 4 against frontal surface 8 of basis 5. For the purpose of achieving said releasable connection between emulator 4 and basis 5, basis 5 and emulator 4 may comprise one or a plurality of connections 7 disposed at frontal surface 8 and back surface 6, respectively.

In preferred embodiments of the present invention, basis 5 may comprises approximately 20% of a total volume of the prosthesis.

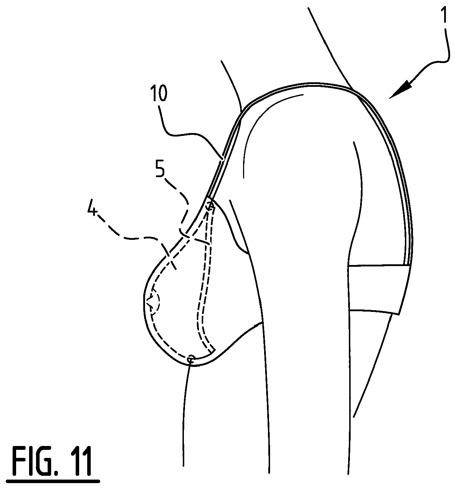

In preferred embodiments of the present invention, engagement surface 9 of basis 5 is further configured to exert a therapeutic compressive force on wound area 3 of the chest portion of user 1. Such a therapeutic compressive force on wound area 3 may be achieved by using a tensioner 10, which is depicted in Figure 11, to press engagement surface 9 against wound area 3 with a predetermined therapeutic compressive force. Tensioner 10 will be further elucidated below with reference to Figure 11. More in particular, engagement surface 9 may exert such a compressive force on wound area 3 by comprising a shape that complements the wound area 3. As is illustrated in Figure 6, basis 5 may comprise an engagement surface 9 having a specific texture that matches a very specific surface area of wound area 3, for example at a specific point in time during a recovery process of user 1 following mastectomy. In these embodiments, the shape of engagement surface 9 may be based on a 3D scan of wound area 3.

In the sense of the present disclosure the engagement surface 9 "complementing" wound area 3 should be understood as engagement surface 9 being shaped and dimensioned to substantially abut wound area 3 when in an engaged state therewith. As such, engagement surface 9 comprises a shape that is at least partially inverse shape of wound area 3.

In addition or alternatively, basis 5 is preferably air permeable so that ambient air may pass through basis 5 and aerate wound area 3, which is considered advantageous as it promotes a healing process of wound area 3 while simultaneously enhancing user comfort while using the prosthesis. As such, basis 5 may comprise a material that is permeable to air and/or may comprise one or more air permeable pores. In preferred embodiments, basis 5 and in particular engagement surface 9 thereof comprises polyester and polyurethane, in particular polyester or polyethylene terephthalate filled with polyurethane.

As is best shown in Figure 4 and Figure 5, back surface 6 of emulator 4 and frontal surface 8 of basis 5 may comprise one or more connections 7. The connections 7 may be of the pin-hole type, wherein back surface 6 and frontal surface 8 each comprise at least one of a hole and a pin or vice versa. In particular, the connections 7 may comprise interlocking press studs or snap fasteners disposed at each one of back surface 6 and frontal surface 8. Such press studs or snap fasteners are preferably disc-shaped and manufactured from a plastic material. Alternatively, connections 7 may be of the hook-and-loop type disposed on the two respective surfaces. However, in more preferred embodiments, the connections 7 are magnetic connections 7, wherein a magnet is disposed on or embedded either one of back surface 6 and frontal surface 8 and an additional magnet, or a magnetically susceptible material such as iron, is disposed on an other one of back surface 6 and frontal surface 8. Moreover, the skilled person will recognise that other types of connections 7 that are easily closed or released by user 1 or a medical professional may be used; as well as combinations of the aforementioned types of connections 7.

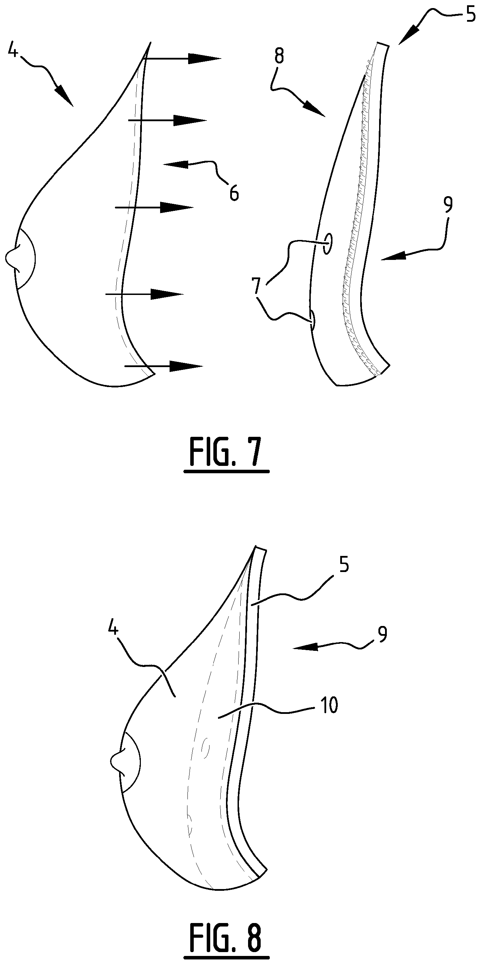

In embodiments of the present invention wherein a plurality of connections 7 is used, the connections may be arranged in pattern designed to assist the user with recognising a proper alignment of basis 5 with emulator 4 when releasably connecting the former with the latter. In particular, the pattern may exhibit an eccentric and/or non-circular shape, wherein at least one of the connections 7 may be arranged at distance relative to a center of the pattern that is different from a distance of at least one other one of the connections 7 relative to the center of the pattern. Figures 4 and 5 in particular illustrate an exemplary embodiment of such configurations for connections 7, which may assist user 1 in connecting emulator 4 to basis 5 with a correct alignment between the two respective components, as is furthermore illustrated in Figure 7.

Figure 8 shows breast prosthesis in accordance with the present invention in an assembled state, wherein emulator 4 has been releasably connected to basis 5 as illustrated in Figure 7. In this state, there exists an interface delineated by the dashed line in Figure 7 between basis 4 and 5 emulator. This interface extends over at least a portion of frontal surface 8 of basis 5 and back surface 6 of emulator 4. While Figure 8 shows the interface extending over approximately the entirety of frontal surface 8 and back surface 6, some embodiments of the present invention may comprise one or more intermediate spaces between the two respective surfaces. These intermediate spaces may help reduce the weight of the prosthesis as a whole, assist in allowing ambient air to pass through basis 5 and aerate wound area 3, or may be present for other design considerations.

Preferred embodiments of a prosthesis in accordance with the present invention may furthermore comprise at least one hook-and-loop fastener arranged along at least a part of the outer circumference of the interface between basis 5 and emulator 4. Such a hook-and-loop fastener arranged at this location may, in addition to or alternatively for the connections 7, serve as a means for releasably coupling emulator 4 to basis 5, as well as for preventing a relative rotation or displacement between these respective two components. Likewise, frontal surface 8 and back surface 6 may exhibit mutually complementary shapes, and in particular mutually interlocking shapes, for this same purpose.

The present invention furthermore relates to a method for applying a prosthesis as described above. According to one general aspect of the present invention, said method comprises providing a basis 4 exhibiting any of the features as described above and having an engagement surface 9 that is configured to engage a chest portion with a wound area 3 of a user 1. According to an other general aspect of the present invention, said method furthermore comprises providing an emulator 4 exhibiting any of the features as described above, wherein the emulator 4 is configured to be releasably connected to the basis 5 and comprising an outer contour emulating a breast 2 of the user 1.

As described above, basis 5 is configured to engage a chest portion comprising a wound area 3, in particular by means of engagement surface 9. During a recovery process following a mastectomy, wound area 3 may undergo changes due to the formation of scar tissue and possibly the occurrence of edema, seroma or radiation fibrosis. Such changes may occur in conjunction with a change in locations on wound area 3 experienced to be sensitive or painful by user 1 as the recovery process progresses. Moreover, in embodiments of the present invention wherein engagement surface 9 is configured to exert a therapeutic compressive force on wound area 3, an adjustment in therapeutic compressive force in terms of magnitude and/or force distribution may be necessary to ensure an optimal recovery process while simultaneously maximising comfort for user while wearing the prosthesis.

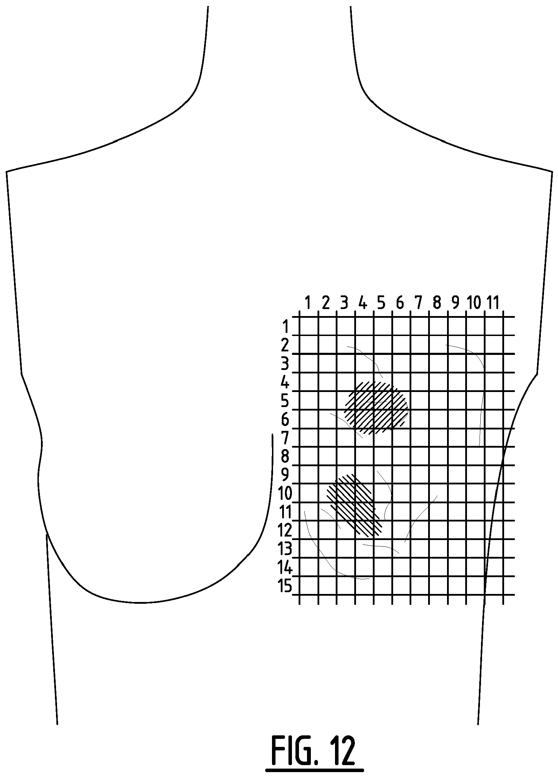

In embodiments wherein engagement surface 9 is based on 3 wound area, for example by being based on a 3D scan thereof, engagement surface 9 may comprise a shape that is at least partially an inverse of a shape of wound area 3 and therefore complements wound area 3. In more preferred embodiments of the present invention, engagement surface 9 moreover comprises at least one of a recess and a protrusion that deviates from said inverse of a shape of wound area 3 to define a predetermined therapeutic pressure profile to be exerted on wound area 3. These embodiments will be elucidated below with reference to Figure 12.

To account for the above needs, the method according the present invention may furthermore advantageously comprise providing a replacement basis 5 for basis 5 after a first periodic interval, in particular a first periodic interval corresponding to a progress in wound healing of the wound area 3 during the recovery process of user 1. Such a replacement basis 5 may comprise an engagement surface 9 that has been 'updated' relative to the previously used engagement surface 9, for example in terms of size, shape or material properties, based on the progress in healing of wound area 3. In particular, the engagement surface may be 'updated' with respect to the aforementioned at least one of a recess and a protrusion to thereby redefine the aforementioned therapeutic pressure profile. Due to (replacement) basis 5 and emulator 4 being releasably coupled, emulator 4 may be advantageously used in conjunction with replacement basis 5 without a need for replacing emulator 4.

In an advance state of or after the recovery process following a mastectomy, wound area 3 may be (almost) completely healed and relatively stable. Consequently, the need for replacing basis 5 relatively frequently for the above described reasons may be diminished or entirely absent. Nevertheless, emulator 4 may be subject to wear and tear during a prolonged period of usage of the prosthesis. Alternatively, a remaining, non-amputated breast 2 of user 1 may experience a change in shape over time, resulting in a mismatch of a contour of remaining, non-amputated breast 2 with the outer contour of emulator 4. To account for at least one or both of these issues, the method according to the present invention may furthermore comprise providing at least one replacement emulator 4 for emulator 4 after a second periodic interval.

As described above, the method according to the present invention may comprise providing a replacement emulator 4 and/or replacement basis 5 for emulator 4 and/or basis 5, respectively. In accordance with an other aspect of the method according to the present invention, providing (replacement) basis 5 may comprise performing a 3D scan of wound area 3 of to obtain a digital model of wound area 3. Basis 5, and in particular engagement surface 9 thereof, may be provided based on this digital model of wound area 3. As such, it is possible to obtain a basis 5 comprising a specific texture that matches a very specific surface area of wound area 3, and thus closely matches the unique characteristics of the chest portion and the wound area 3 thereon, to achieve both maximum user comfort and/or optimal exertion of therapeutic compressive force on wound area 3.

Moreover, the method according to the present invention may comprise performing a 3D scan of breast 2 of user 1 to obtain a digital model of the breast of the user. Preferably, breast 2 is scanned prior to surgical removal through mastectomy, so that the outer contour of (replacement) emulator 4 accurately emulates said breast 2. Alternatively, the 3D scan may be performed post-mastectomy on a remaining, non-amputated breast 2 so that the outer contour of emulator 4 closely emulates a, preferably mirrored, outer contour of said breast 2.

Regardless of whether emulator 4 is based on 3D scan of breast 2, the method according to the present invention may furthermore comprise printing emulator 4 by means of a 3D printer. Alternatively, the method may comprise printing a mold for emulator 4 by means of a 3D printer and subsequently providing emulator 4 by molding a curing material in the mold, after which emulator 4 may be retrieved from the mold. Providing emulator 4 by means of a mold is the preferred option when the material for emulator 4 exhibiting the desired characteristics (e.g. in terms of density and/or elasticity) can not be 3D printed directly.

In embodiments of the present invention wherein back surface 6 of emulator 4 and frontal surface 8 of basis 5 comprise one or more magnetic connections 7, one or more magnets or magnetically susceptible materials may be disposed at locations where emulator 4 is to be 3D-printed or molded in the mold using the curing material. The one or more magnetic connects may be arranged in a pattern designed to assist the user with recognising a proper alignment of basis 5 with emulator 4 when connecting the former with the latter, as has been described above.

Moreover, the method in accordance with the present invention may comprise arranging at least one hook-and-loop fastener at basis 5 and emulator 4, in particular along a circumference of an interface between these respectively two components when releasably connected to one another.

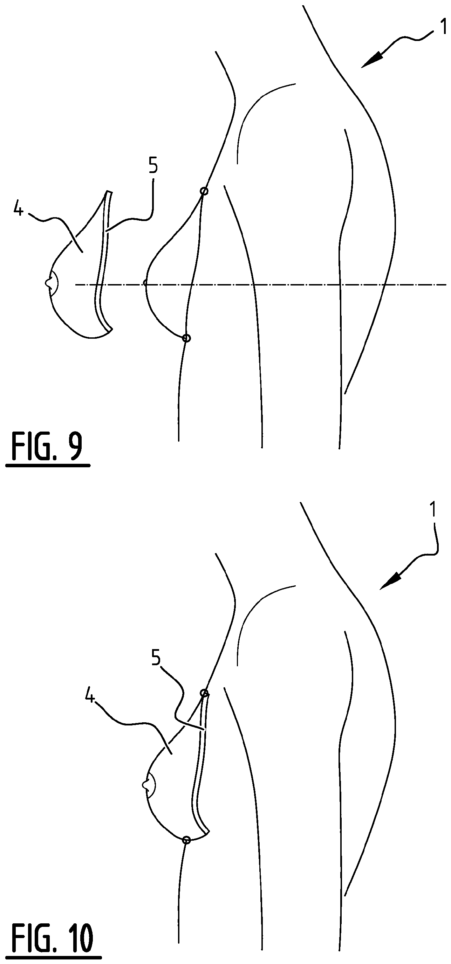

The method according to the present invention may furthermore comprise releasably coupling emulator 4 to basis 5 as well as applying the prosthesis to user 1 as illustrated in Figure 9 and Figure 10.

Figure 11 shows the prosthesis likewise depicted in Figure 10 in conjunction with an exemplary embodiment of a tensioner 10 in accordance with the present invention.

Tensioner 10 comprises an elastic material and encloses a part of the body of user 1 and the prosthesis comprising basis 5 and emulator 4. Due to its elastic properties, tensioner 10 generates an elastic force, that is to say a therapeutic compressive force, which is in turn transferred to wound area 3 by engagement surface 9 of basis 5 to achieve the aforementioned therapeutic effects.

Tensioner 10 is preferably dimensioned to comfortably yet securely fit around a body of user 1. Moreover, the physical dimensions and/or its elastic properties of tensioner 10 are selected to generate the aforementioned elastic force having a predetermined magnitude large enough to achieve the aforementioned therapeutic effects promoting healing of wound area 3, yet small enough to not discomfort user 1 or to abate healing of wound area 3. A pressure associated with the compressive force exerted on wound area 3 by engagement surface 9 as a result of the elastic force generated by tensioner 10 may lie within the range of 10 to 35 mmHg (1,3 to 4,6 kPa).

The skilled person will recognise that tensioner 10 can comprise various alternative implementations in accordance with various embodiments of the present invention. In the exemplary embodiment of Figure 11, tensioner 10 essentially covers the prosthesis including emulator 4. Alternatively, tensioner 10 may be integrated with basis 5, with tensioner 10 leaving emulator 4 exposed in an assembled state.

Tensioner 10 may moreover comprise a plurality of alternative materials, including natural and synthetic rubbers, as well as fabrics used in therapeutic compressive garments known in the Art. Moreover, tensioner 10 may be constituted by a (compressive) bra, whether or not of the type used for therapeutic purposes in the Art.

Tensioner 10 may be comprised by the assembly or the kit-of-parts in accordance with the present invention and as defined in the appended claims.

Figure 12 illustrates a digital model wound area 3 of user 1 superimposed by a grid. In accordance with a method for applying a prosthesis in accordance with the present invention, a three-dimensional scan of wound area 3 may be made to obtain the digital model of wound area 3. As can be discerned from Figure 12, the digital model of wound area 3 comprises an essentially irregular surface including various bumps and depressions.

The exemplary digital model of wound area 3 depicted in Figure 12 moreover comprises two shaded areas, one being situated higher on the vertical axis of the superimposed grid than the other. These two shaded areas of the exemplary digital model respectively represent areas of wound area 3 of a first type and a second type, both of which will be elucidated here below.

Wound area 3 may include one or more areas of a first type that are susceptible to medical complications, including local secondary lymphedema, postoperative seroma and radio-induced fibrosis. In the exemplary digital model depicted in Figure 12, such an area of the first type is represented by the first shaded area situated higher on the vertical axis of the superimposed grid. To promote a healing process of such areas, it is desirable to exert a(n) (increased) therapeutic pressure thereon to prevent or abate the aforementioned medical complications.

Wound area 3 may moreover include one or more areas of a second type that are relatively sensitive to any pressure exerted thereon, for example due to the presence of prominent scar tissue, birthmarks or hypersensitive skin portions. In the exemplary digital model depicted in Figure 12, such an area of the second type is represented by the second shaded area situated lower on the vertical axis of the superimposed grid. Exerting pressure on such areas of the second type may induce discomfort or pain and is therefore preferably avoided.

In accordance with a preferred embodiment of a method for applying a prosthesis in accordance with the present invention, these two types of areas are identified in the digital model of wound area 3, for example by user 1 and a medical practitioner. The obtained three-dimensional model depicted in Figure 12, having included therein information with respect to at least one of these two types of areas, is then subsequently used to further determine a shape of engagement surface 9 of basis 5 that is initially based on the three-dimensional scan of wound area 3.

Identifying areas of the first type or the second type in the digital model of wound area 3 may comprise selecting grid spaces or grid points in the grid depicted in Figure 12.

In particular at least one protrusion may be provided at engagement surface 9 that corresponds to the first shaded area of the digital model of Figure 12, which corresponds to an area of the first type of wound area 3. In addition or alternatively, at least one recess may be provided at engagement surface 9 that corresponds to the second shaded area of Figure 12, which corresponds to an area of the second type of wound area 3.

As such, an engagement surface 9 is obtained that defines a specific, predetermined therapeutic pressure profile to be transferred to wound area 3. Said predetermined therapeutic pressure profile, while substantially corresponding to the physical dimensions of wound area 3, moreover promotes an optimal healing process of areas of the first type of wound area 3 and simultaneously transfers very little or no therapeutic pressure to sensitive areas of the second type of wound area 3 to optimise user comfort.

The above described embodiments are intended only to illustrate the invention and not to limit in any way the scope of the present invention. Based on the above description of specific and particular embodiments of the present invention, the scope of protection according to the appended claims has been substantiated, whereas clearly additional and/or alternative embodiments are possible, feasible and potentially desirable within the scope of protection according to those appended claims. Features of the specific embodiments can not and should not result in limitation of the scope of protection thereto, unless defined in independent claims, whereas even the features defined in the appended independent claims can be replaced by obvious alternatives. Accordingly, it should be understood that where features mentioned in the appended claims are followed by reference signs, such signs are included solely for the purpose of enhancing the intelligibility of the claims and are in no way limiting on the scope of the claims. The scope of the invention is defined solely by the following claims.

## Claims

1. A prosthesis, comprising:
- a basis having an engagement surface configured to engage a chest portion with a wound area of a user;
- an emulator configured to be releasably connected to the basis and comprising an outer contour emulating a breast of the user; and
- wherein the engagement surface of the basis comprises a shape that substantially complements the wound area to transfer a compressive force to the wound area.

2. Prosthesis according to claim 1, wherein the shape of the engagement surface that complements the wound area is based on a three-dimensional, 3D, scan of the wound area; and
wherein said engagement surface moreover comprises at least one of a recess and a protrusion relative to the shape of the engagement surface, that otherwise complements the wound area, to define a predetermined pressure profile to be transferred on the wound area.

3. Prosthesis according to claim 1 or 2, wherein the basis is air permeable to allow aeration of the wound area.

4. Prosthesis according to any one of the foregoing claims, wherein a shape of the outer contour emulating the breast of a user is based on a 3D scan of a breast of the user.

5. Prosthesis according to any one of the foregoing claims, wherein the basis comprises at least one material from a group of materials comprising polyester and polyurethane.

6. Prosthesis according to any one of the foregoing claims, wherein the emulator is configured to be releasably connected to the basis by means of at least one releasable connection disposed at the basis and the emulator.

7. Prosthesis according to claim 6, wherein the emulator is configured to be releasably connected to the basis by means of a plurality of connections disposed at the basis and the emulator.

8. A method for applying a prosthesis, the method comprising:
- providing a basis having an engagement surface configured to engage a chest portion with a wound area of a user, wherein the engagement surface of the basis comprises a shape that substantially complements the wound area to transfer a compressive force to the wound area; and
- providing an emulator configured to be releasably connected to the basis and comprising an outer contour emulating a breast of the user.

9. Method for applying a prosthesis according to claim 8, wherein the step of providing the basis further comprises:
- performing a three-dimensional, 3D, scan of the wound area of the chest portion of the user to obtain a digital model of the wound area;
- identifying, in the digital model of the wound area, at least one of:
- an area requiring a decreased or absent pressure to be transferred thereto, and
- an area requiring an increased pressure to be transferred thereto; and
- providing, relative to the shape of the engagement surface that otherwise complements the wound area, at least one of:
- a recess corresponding to the area requiring a decreased or absent pressure to be transferred thereto, and
- a protrusion corresponding to the area requiring an increased pressure to be transferred thereto.

10. Method for applying a prosthesis according to claim 8 or 9, further comprising providing at least one replacement basis after a first periodic interval.

11. Method for applying a prosthesis according to claim 10, wherein the first periodic interval corresponds to a progress in wound healing of the wound area.

12. Method for applying a prosthesis according to any one of the foregoing claims 8 - 11, further comprising providing at least one replacement emulator after a second periodic interval.

13. Method for applying a prosthesis according to any one the of foregoing claims 8 - 12, wherein the step of providing the emulator further comprises:
- performing a 3D scan of a breast of the user to obtain a digital model of the breast of the user; and
- providing the emulator based on the digital model of the breast of the user.

14. Method for applying a prosthesis according to claim 13, wherein providing the emulator based on the digital model of the breast comprises at least one of:
- printing, by means of a 3D printer, the emulator; and
- printing, by means of a 3D printer, a mold of the emulator and subsequently providing the emulator by molding a curing material in the mold.

15. Kit-of-parts, comprising a basis and an emulator of the prosthesis according to any of claims 1 - 7.
